# EUROPEAN PATENT SPECIFICATION

(11) **EP 1 600 099 B1**
(45) Date of publication and mention of the grant of the patent: **04.12.2013**
(21) Application number: 03768226.7
(22) Date of filing: 25.12.2003
(51) Int. Cl.: A61B 1/00

(54) **COLLECTING DEVICE OF CAPSULE TYPE MEDICAL UNIT AND ITS COLLECTING METHOD**
SAMMELVORRICHTUNG FÜR KAPSELARTIGE MEDIZINISCHE EINHEIT UND SAMMELVERFAHREN
DISPOSITIF ET PROCEDE SERVANT A RECUEILLIR DES CAPSULES MEDICALES

(30) Priority: 06.03.2003 JP 2003060152
(43) Date of publication of application: 30.11.2005
(73) Proprietor: Olympus Corporation, Tokyo 151-0072 (JP)
(72) Inventor: TAKIZAWA, Hironobu, Hachioji-shi, Tokyo 193-0844 (JP); YOKOI, Takeshi, Hino-shi, Tokyo 191-0042 (JP)
(74) Representative: von Hellfeld, Axel
(86) International application number: PCT/JP2003/016693
(87) International publication number: WO 2004/078036

(56) References cited:
- WO-A-02/082979
- WO-A-02/100256
- DE-A1- 3 001 683
- DE-A1- 10 103 382
- JP-A- 5 007 573
- JP-A- 2003 019 111
- JP-A- 2003 260 026
- US-A- 3 540 433
- US-A- 4 172 446
- US-A- 4 445 235
- US-A- 5 337 426
- US-A1- 2002 198 439
- US-A1- 2003 020 810
- US-B1- 6 324 418

## Description

### Technical Field

The present invention relates to a device and a method for retrieving a medical capsule for use in medical examination of the human body taken into and discharged from within the human body.

### Background Art

Japanese Unexamined Patent Application Publication No. 11-225996 discloses, as a conventional medical capsule, a swallowing type capsule intracorporeal-information detecting unit which stays within the human body for a while to collect biomedical information. With biomedical information captured, the capsule intracorporeal-information detecting unit is discharged from within the human body after passing through the digestive tracts.

The discharging and cleaning process of the capsule intracorporeal-information detecting unit is mentioned but no idea is presented of how to detect and retrieve the discharged capsule in the above-referenced conventional art, thus the medical capsule is impractical.

PCT International Patent Publication WO 03/005877 A2 discloses a device and a method for examining a body tract. This device is reduced in size from the initial state thereof a predetermined period of time later so that the device may pass through a narrowed area in a tract of the body.

Publication WO 03/005877 A2 discloses a monitoring technique to monitor the location of the device, but fails to state a mechanism to easily retrieve the device discharged from the tract of the body.

WO 2002/082979 discloses an in vivo vehicle to be navigated by extracorporeal devices, including electromagnetic field generators and electromagnetic field detectors which act on a magnet of the in vivo vehicle and detect a position of the in vivo vehicle, respectively.

JP 2005/00757 discloses a tablet-type sensor to be swallowed by a patient for diagnostic purposes. The sensor comprises a chemical sensor or a biosensor and a controller and memory, and can be recovered after being discharged from the patient.

DE 10103382 A1 discloses a device for catching stool samples, the device comprising a catch bowl having multiple apertures that are distributed amongst the bottom of the catch bowl. Such design of the catch bowl essentially allows catching only the stool. Urine that is possibly caught in the catch bowl may immediately escape through the apertures. DE 10103382 A1 however fails to teach how to facilitate detection or retrieval of a medical capsule from waste discharged from the tract of the body.

US 3540433 discloses a feces strainer for use in easily and simply collecting a stool specimen. By its ability to pass liquid constituents in a substantially nonadhesive manner it is intended to facilitate the removal of solid feces specimen from the strainer. Detection or retrieval of a medical capsule from waste discharged from the tract of the body is however not discussed in US 3540433.

The present invention has been developed in view of this situation, and it is an object of the present invention to provide a medical capsule retrieval device that allows a medical capsule to be easily retrieved after being discharged from within the human body, and a method of retrieving the medical capsule.

### Disclosure of Invention

A medical capsule retrieval device according to claim 1 is provided. A retrieval method for retrieving a medical capsule device according to claim 10 is provided. The medical capsule retrieval device includes a detecting means for detecting a medical capsule discharged from within the human body comprising receiving means that detects the radio wave signal emitted from the medical capsule, and a catching means for catching the medical capsule, said catching means comprising a net.

### Brief Description of the Drawings

Fig. 1A to Fig. 4D relate to a first example not forming part of the present invention.
Fig. 1A illustrates configuration of a capsule type medical system in use in accordance with the first example, and Fig. 1B illustrates a personal computer, connected to an extra-corporeal unit, for displaying and storing biomedical information in the system;
Fig. 2 illustrates the internal structure of a capsule type endoscope;
Fig. 3A illustrates the structure of a distal end of a retrieval tool in the first example, and Fig. 3B illustrates the structure of a distal end of a retrieval tool in a modification of the first example;
Figs. 4A-4D illustrate the operation of the retrieval tool in accordance with the first example;
Fig. 5 to Fig. 9 relate to a second example.

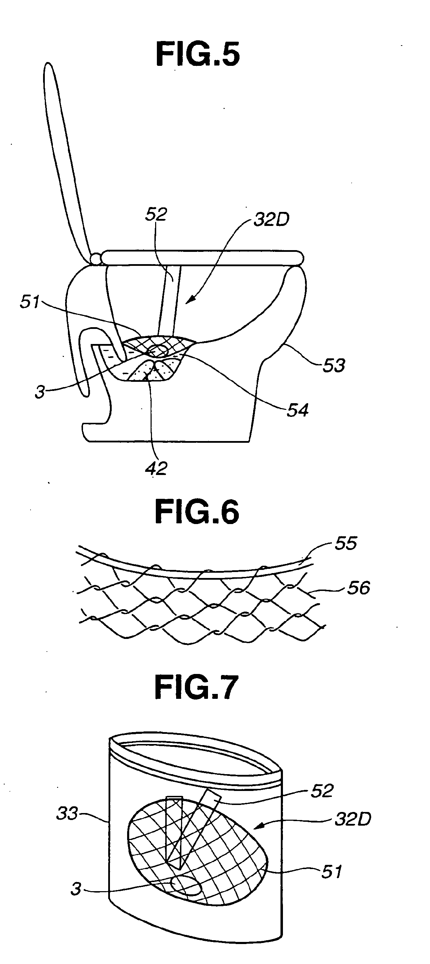

Fig. 5 illustrates the operation of a retrieval tool in use in accordance with the second example;
Fig. 6 illustrates in enlargement a retrieval net;
Fig. 7 illustrates a retrieved capsule type endoscope together with the retrieval tool introduced into a bag;

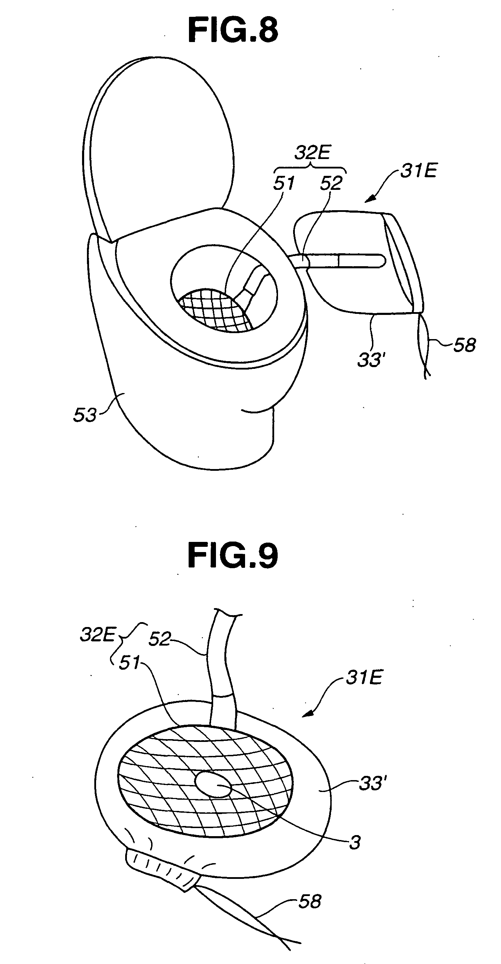

Fig. 8 illustrates the operation of a retrieval tool in use of a modification of the second example;
Fig. 9 illustrates a bag inside out with a top closing string fastened;

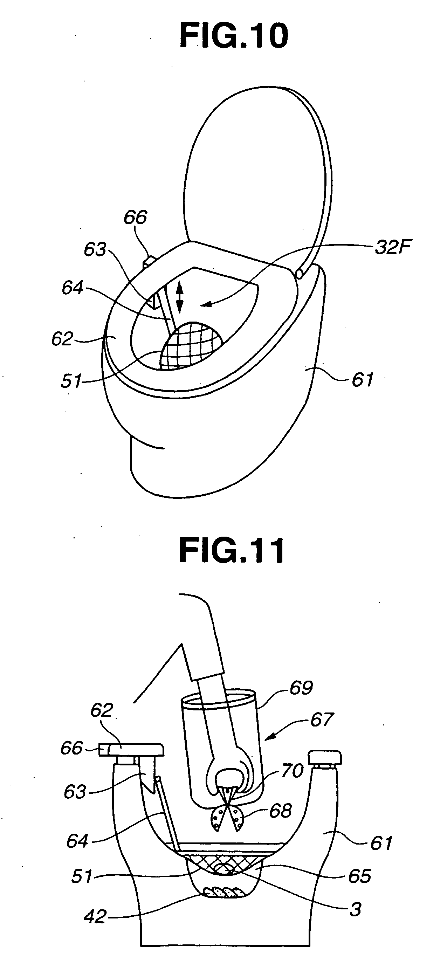

Fig. 10 to Fig. 14 relate to a third example.
Fig. 10 illustrates a retrieval tool installed on a toilet bowl in accordance with the third example;
Fig. 11 illustrates a retrieval operation to retrieve the capsule type endoscope using the retrieval tool;

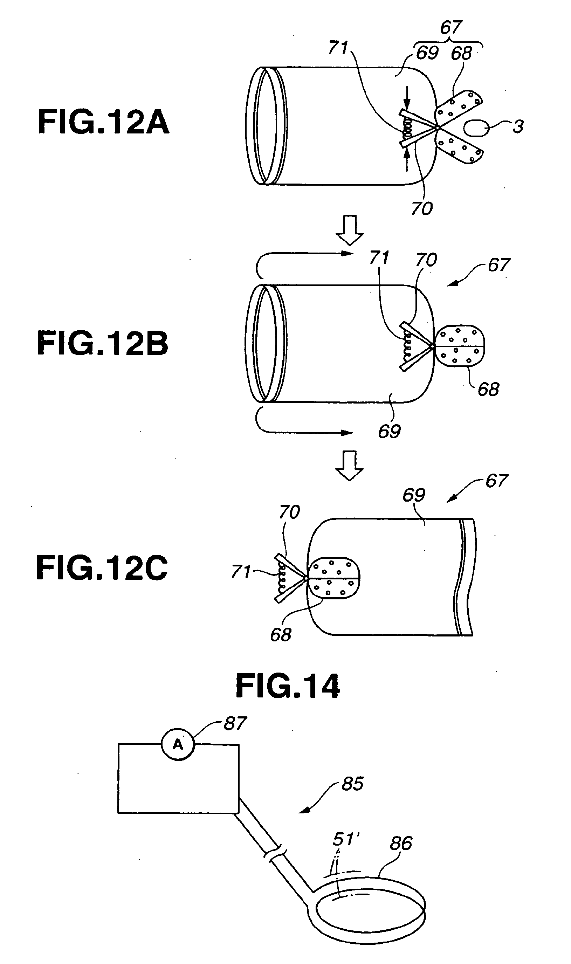

Figs. 12A-12C illustrate a retrieval and storage operation in which the capsule type endoscope is retrieved using a container having a bag with cup-shaped catches attached thereto;

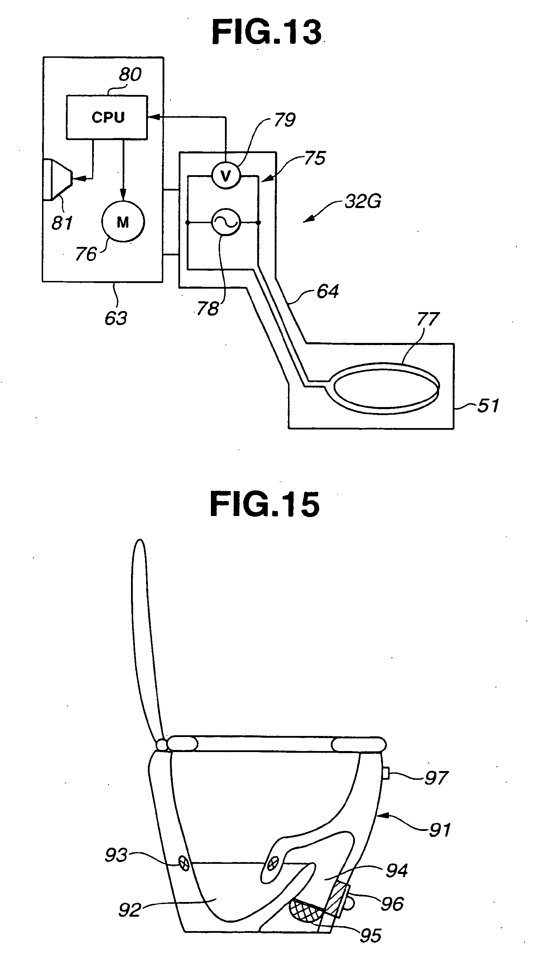

Fig. 13 illustrates an electrical system of a retrieval tool having a sensor in accordance with a first modification of the third example;
Fig. 14 illustrates a magnetic sensor of a second modification in accordance with the third example; Fig. 15 to Fig. 18 relate to a fourth example.
Fig. 15 illustrates configuration of a retrieval device in accordance with the fourth example;

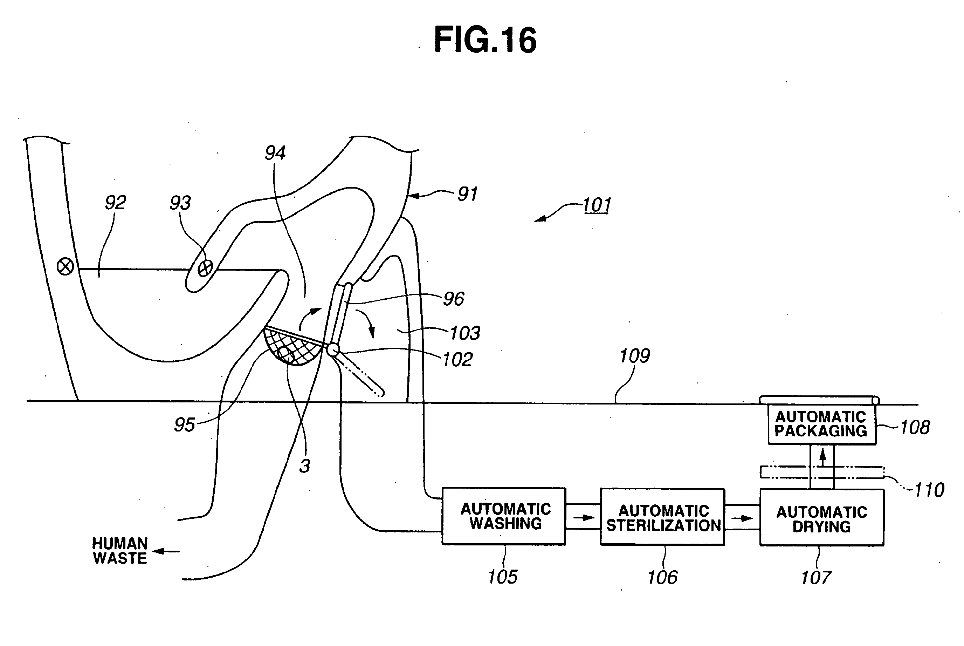

Fig. 16 illustrates a retrieval device in accordance with a second modification of the fourth example;

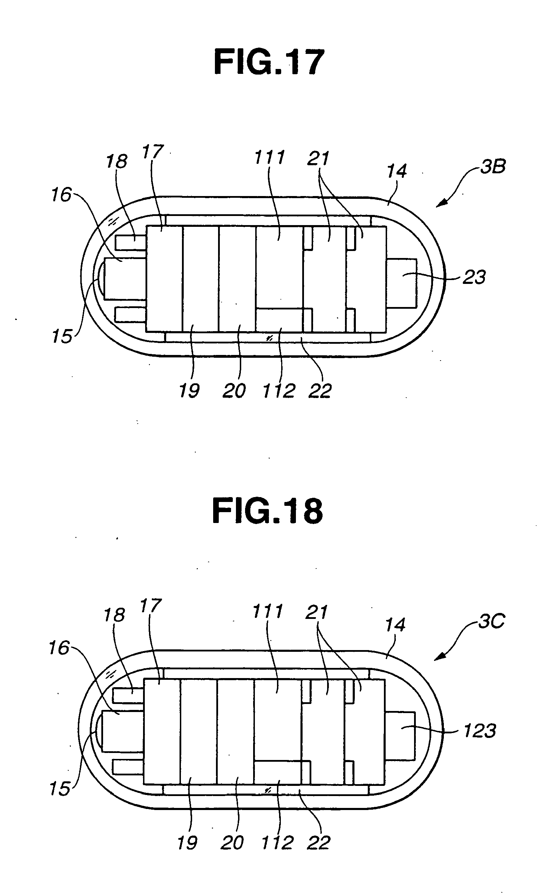

Fig. 17 illustrates the internal structure of the capsule type endoscope in accordance with a third modification of the fourth example;
Fig. 18 illustrates the internal structure of the capsule type endoscope in accordance with the third modification of the fourth example;

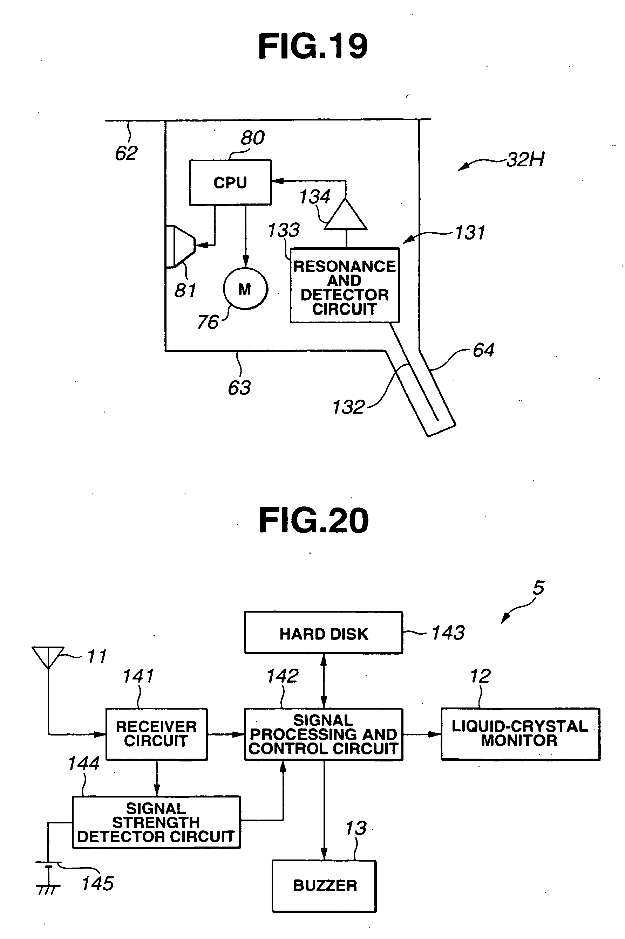

Fig. 19 and Fig. 20 relate to a first embodiment of the present invention.
Fig. 19 illustrates an electrical system of a retrieval tool in accordance with the first embodiment of the present invention; and
Fig. 20 is a block diagram illustrating an extra-corporeal unit in accordance with a first modification of the first embodiment of the present invention.

### Best Mode for Carrying Out the Invention

The examples and embodiments of the present invention are discussed below with reference to the drawings.

### First Example

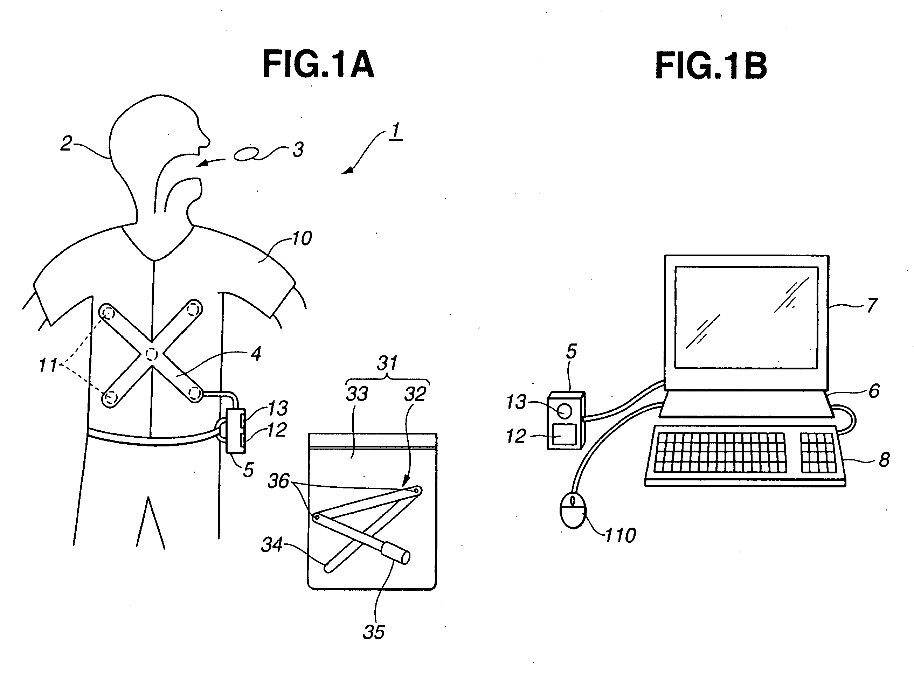

A first example not forming part of the present invention will now be discussed with reference to Fig. 1A through Fig. 4D.

As shown in Fig. 1A, a subject 2 swallows a capsule type endoscope (hereinafter simply referred to as a capsule) 3 as a medical capsule through the mouth. When passing through a bodily tract, the capsule 3 picks up an optical image of the wall of the tract, and then transmits the corresponding image signal on a radio wave. A capsule type medical system 1 includes the capsule 3, an antenna unit 4 being provided outside the subject 2 and for receiving the signal transmitted from the capsule 3, an extra-corporeal unit 5 (mounted outside the subject 2) having a function for storing the image, and a capsule retrieval device (hereinafter simply referred to as a retrieval device) 31 for retrieving the capsule discharged from within the subject body.

As illustrated in Fig. 1B, the extra-corporeal unit 5 is detachably connected to a personal computer (hereinafter PC) 6. The PC 6 captures the image stored in the extra-corporeal unit 5, and stores the image in an internal hard disk or displays the image on a display 7. A keyboard 8 is connected to the PC 6 to input data, etc.

As shown in Fig. 1A, to perform an endoscopic examination with the capsule 3, the subject 2, wearing a jacket 10 with the antenna unit 4 having a plurality of antenna elements 11 mounted thereon, swallows the capsule 3. The capsule 3 picks up an image of the internal tract of the subject 2, and transmits the corresponding image signal from a built-in antenna to the antenna unit 4. Upon receiving the image signal, the extra-corporeal unit 5 connected to the antenna unit 4 stores the captured image. The extra-corporeal unit 5 may be mounted on a belt of the subject 2 using a detachable hook.

The extra-corporeal unit 5 may have a box-like configuration, for example, and has, on the front section thereof, a liquid-crystal monitor 12 for presenting an image thereon and a buzzer 13 for alerting.

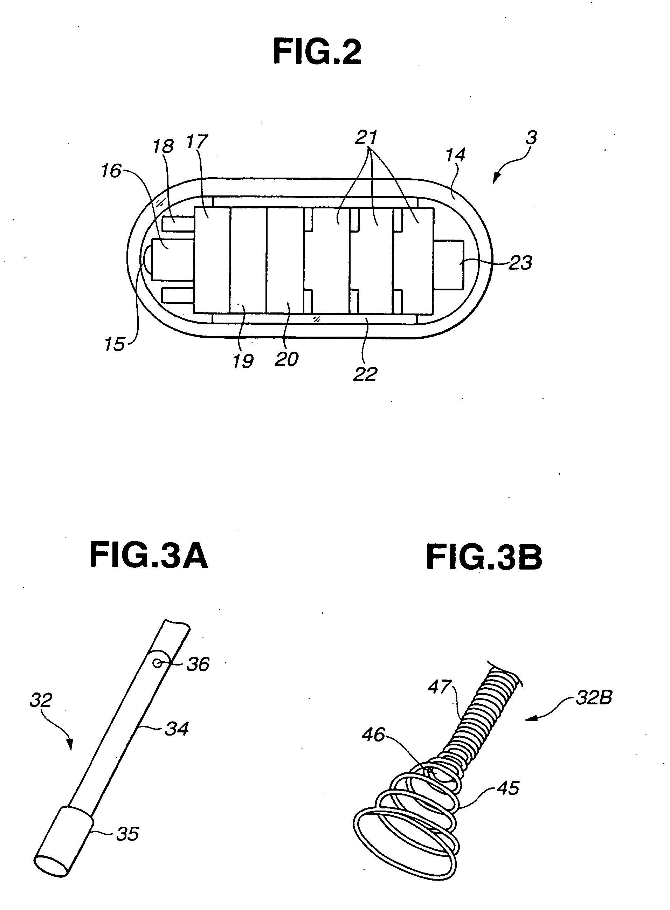

As illustrated in Fig. 2, the capsule 3 is a watertight transparent housing 14 including a center cylinder portion closed by semi-spherical end portions. Arranged inside and near an image pickup end of transparent housing 14 is an objective lens 15 mounted in the center of a lens frame 16. Arranged at the focusing position of the objective lens 15 is a CMOS imager 17 functioning as an image pickup device.

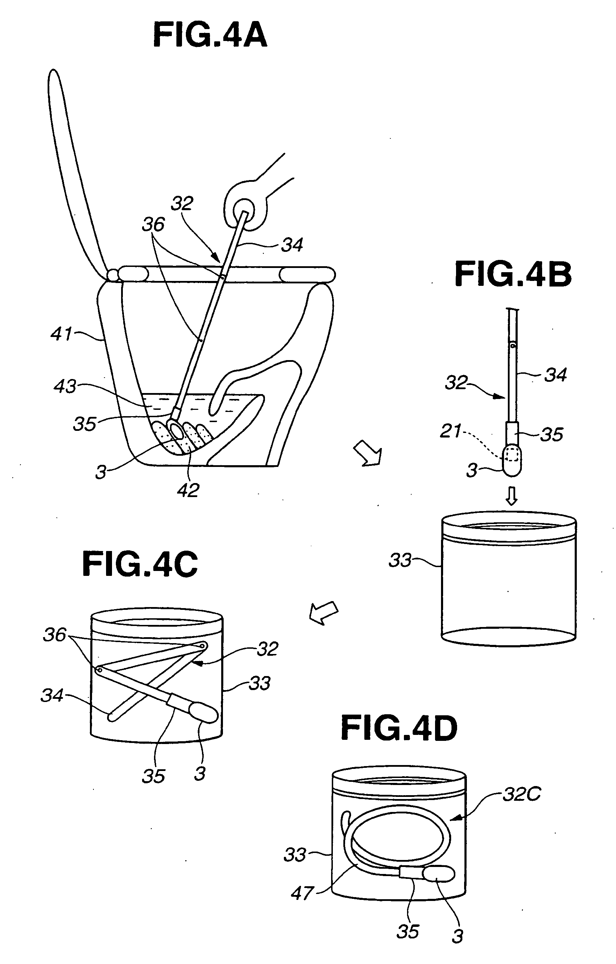

White LEDs 18 are arranged around the objective lens 15 as an illumination system. Arranged behind the CMOS imager 17 inside a transparent inner cylinder member 22 in the transparent housing 14 are a control circuit (or a processing circuit) 19 for driving the CMOS imager 17 and generating an image signal from the image captured by the CMOS imager 17, a communication circuit 20 for modulating the image signal and transmitting the modulated signal, and button batteries 21 for feeding power to the control circuit With this arrangement, the subject can use the retrieval tool 32C in the straight state thereof to retrieve the capsule 3. When the capsule 3 is stored subsequent to the retrieval operation, the retrieval tool is curved in a loop to be introduced into the bag 33 as shown in Fig. 4D. The bag 33 is then closed for storage.

The retrieval tool of the second modification of the first example is thus easily handled.

In the above discussion, the magnet 35 is attached to the retrieval tool 32 side. Alternatively, the magnet may be attached to the capsule 3 side, and the magnetic material may be arranged in the retrieval tool 32 side, or magnets may be attached to both the capsule 3 side and the retrieval tool 32 side. The magnet may be one of a permanent magnet and an electromagnet.

A third modification of the first example is now discussed. The PC 6 and the display 7 in the capsule type medical system 1 shown in Fig. 1 have at least one of the following plurality of functions.
(A) Operation means for controlling image replay settings (controlling functions such as replay of image, replay of image in reverse, fast forward, rewinding, stop, change of replay speed) are available. The operation means includes a button, a slide bar, a pulldown menu, etc. capsule 3 by attraction. For the convenience of use in the first example, the rod 34 of the retrieval tool 32 is hinged at a plurality hinge portions 36 in the longitudinal direction thereof.

The magnet may be a permanent magnet such as an inexpensive ferrite magnet, a high-magnetic force neodymium magnet, or a cobalt magnet, or may be an electromagnet which is turned on during a retrieval operation only.

A medical staff may hand over the retrieval tool 32 in the enclosure bag 33 to the subject 2 who undergoes a medical check by the capsule 3 as shown in Fig. 1A.

The enclosure bag 33 is used not only to store the retrieved capsule 3 as shown in Fig. 4C subsequent to the retrieval of the capsule 3, but also to store the soiled retrieval tool 32 in the enclosure bag 33.

The retrieval operation of the retrieval device 31 in accordance with the first example will now be discussed with reference to Figs. 4A through 4D.

The subject who has swallowed the capsule 3 searches waste 42 for the capsule 3 using the retrieval tool 32 of the first example when the subject defecates about 8 hours or more after the swallowing of the capsule 3. The subject checks to see whether or not the capsule 3 is discharged together.

When the subject 2 defecates in a toilet bowl 41 as shown in Fig. 4A, the capsule 3 may be discharged together with the waste 42. The capsule 3 contains the button battery 21 that is attracted by a magnetic force.

As shown in Fig. 4A, holding the proximal end of the retrieval tool 32, the subject 2 places the distal end of the retrieval tool 32 close to the waste 42. The magnet 35 arranged at the distal end of the retrieval tool 32 attracts the button battery 21, thereby detecting the presence of the capsule 3 in the waste 42. The capsule 3 is attracted to and is thus held to the magnet 35.

To facilitate the detection of the magnetic force of the capsule 3, the toilet bowl 41 is preferably a nonmagnetic material free from the effect of magnetic force. The same is true in other examples and embodiments where magnetic force is used for detection.

In accordance with the first example, the magnet 35, attached to the distal end of the retrieval tool 32, attracts the magnetic metal (the battery or circuit) or a magnet in the capsule 3. The capsule 3, if present in the waste 42, is thus detected and retrieved.

If the retrieval tool 32 attracts the capsule 3 at the distal end thereof, the user swings the distal end of the retrieval tool 32 in water 43 in the toilet bowl 41 to shake sticking waste 42 off the capsule 3, and raises the capsule 3 from the toilet bowl 41.

Referring to Fig. 4B, the subject introduces the capsule 3 and the retrieval tool 32 into the enclosure bag 33 for retrieval through the mouth thereof.

As shown in Fig. 1A, the retrieval tool 32 is stored in the folded state thereof in the bag 33 before use. As shown in Fig. 4C, the retrieval tool 32 in the folded state thereof and the capsule 3 are stored after use. The subject closes the mouth of the bag 33 for storage. If the capsule 3 is not yet discharged, the retrieval tool 32 may be left in the toilet bowl 41. Alternatively, the subject may stores the capsule 3 each time of use.

The subject returns the closed bag 33 to a manufacture or a hospital. Alternatively, the subject carries the capsule 3 with him when he returns the extra-corporeal unit 5 to a hospital. A person in charge in the hospital sends the retrieved capsules 3 in lot to a manufacturer.

The first example has the following advantages.

In accordance with the first example, the retrieval tool 32 is simple and light-weight, and the subject retrieves the capsule 3 at any convenient place outside a hospital, such as at home or place of work.

If the capsule 3 is battery powered, a low-cost retrieval tool 32 works without the need for any particular retrieval device in the capsule 3.

Fig. 3B illustrates a distal end of a retrieval tool 32B in accordance with a first modification of the first example.

As shown in Fig. 3B, the retrieval tool 32B has a coil spring 45 having a diameter becoming larger toward the distal end thereof. A spherical magnet 46 is attached to the base portion of the coil spring 45, for example.

Using the retrieval tool 32B, the user attracts the capsule 3 with the magnet 46 and receives the capsule 3 in the coil spring 45. In this modification, the capsule 3 is easily retrieved in the stably held position thereof.

The capsule 3 is thus reliably retrieved.

A second modification of the first example is discussed below. Fig. 4D illustrates a retrieval tool 32C of the second modification of the first example with the capsule 3 in the retrieved state thereof.

As shown in Fig. 3B, the retrieval tool 32C includes a tightly wound coil 47 on a rod portion thereof proximal from the base of the coil spring 45. The tightly wound coil 47 has an appropriate hardness and flexibility. (A rod is illustrated as the tightly wound coil 47 in Fig. 3B.)

The rod portion of the retrieval tool 32C, namely, the tightly wound coil 47 is normally straight as shown, but is flexible enough to be curved.

With this arrangement, the subject can use the retrieval tool 32C in the straight state thereof to retrieve the capsule 3. When the capsule 3 is stored subsequent to the retrieval operation, the retrieval tool is curved in a loop to be introduced into the bag 33 as shown in Fig. 4D. The bag 33 is then closed for storage.

The retrieval tool of the second modification of the first aspect of the present invention is thus easily handled.

In the above discussion, the magnet 35 is attached to the retrieval tool 32 side. Alternatively, the magnet may be attached to the capsule 3 side, and the magnetic material may be arranged in the retrieval tool 32 side, or magnets may be attached to both the capsule 3 side and the retrieval tool 32 side. The magnet may be one of a permanent magnet and an electromagnet.

A third modification of the first example is now discussed. The PC 6 and the display 7 in the capsule type medical system 1 shown in Fig. 1 have at least one of the following plurality of functions.
(A) Operation means for controlling image replay settings (controlling functions such as replay of image, replay of image in reverse, fast forward, rewinding, stop, change of replay speed) are available. The operation means includes a button, a slide bar, a pulldown menu, etc. graphically drawn on a screen.
(B) A plurality of images are concurrently displayed on a plurality of windows. Operation means for controlling image replay settings (controlling functions such as replay of image, replay of image in reverse, fast forward, rewinding, stop, change of replay speed) are available to permit an individual setting on a window-by-window basis. Also available is operation means to control the setting common to all windows at a time.
(C) The user may select a desired image from displayed images, and attaches a marker or a comment to the selected image. The user may present a selection image screen listing only the images having the marker thereon. The user may also present an image and comment screen listing only the image having the comment thereon.
(D) The user may switch a display screen from one state to another. For example, the display screen selectively presents a multi-window screen state, a single-window screen state, a thumbnail display state, a selection image display state, and a comment display state.
(E) The user concurrently opens a plurality of different examination information. The plurality of different examination information may be switched using a tab method. The user clicks a tab using a pointing device 110 (see Fig. 1B), a wheel of the pointing device 110, or a tab key or a cursor key of the keyboard 8.
(F) The user presents the images captured at regular intervals (every predetermined number of images or every predetermined time) from among all images taken. By clicking or double-clicking the pointing device 110 with a pointer on a displayed image, the user shifts the display screen to the single-window screen to replay that image and subsequent images.
(G) The PC 6 and the display 7 include operation means for establishing a communication link with the extra-corporeal unit 5 of the capsule 3.
(H) Subsequent to the start-up of the examination using the capsule 3, the PC 6 issues a communication link establishment request, a communication termination request, an image capture request, and an imaging stop request. The extra-corporeal unit 5 is provided with the same function. The user thus operates the capsule type medical system 1 from either the PC 6 or the extra-corporeal unit 5.
(I) The capsule type medical system 1 has a setting function that modifies various settings in the capsule 3 including an image capturing interval, an exposure condition, and an output of an illumination 18 during the examination using the capsule 3. The settings are entered by operating the keyboard 8 or the pointing device 110.

The extra-corporeal unit 5 further has the following functions.
(A) Display means (such as an LCD, an electric bulletin board, an LED, etc.) is arranged on the extra-corporeal unit 5 to indicate the state of the capsule 3 (remaining battery power, temperature, a communication status, an error status, etc.).
(B) The extra-corporeal unit 5 has a setting function for modifying the settings of the capsule 3 including an image capturing interval, an exposure condition, and an output of an illumination 18. The settings are modified using setting means, such as a button or a switch, arranged on the extra-corporeal unit 5.
(C) The setting means arranged on the extra-corporeal unit 5 has an error prevention mechanism. The error prevention mechanism includes at least one of the following controls listed below.

(a) A plurality of buttons or switches that must be concurrently operated.
(b) A control that must be continuously operated for a long period of time.
(c) Operation means having a cover thereon.
(d) Operation means recessed from a surrounding operation surface.
(e) A plurality of buttons or switches that must be operated in a predetermined sequential order.

In accordance with the third modification, the ease of use of the capsule 3 is improved with a variety of functions incorporated. Examination and diagnosis using the capsule 3 are thus facilitated.

### Second Example

A second example will now be discussed with reference to Figs. 5 through 9. The second example relates to a net type retrieval tool. Fig. 5 illustrates the retrieval tool of the second example of the present invention in use.

As shown in Fig. 5, the retrieval tool 32D includes a retrieval net 51 and a band-like handle 52 with one end thereof connected to the retrieval net 51. After the capsule 3 is caught by the retrieval net 51, the retrieval net 51 is stored together with the capsule 3 in the bag 33 as shown in Fig. 7.

Referring to Fig. 5, the retrieval tool 32D is set so that the circular retrieval net 51 covers a drain hole 54 of a toilet bowl 53. The handle 52 with the one end connected to the retrieval net 51 extends upward along the inner surface of the toilet bowl 53. The upper portion of the handle 52 may be folded back along the upper edge of the toilet bowl 53 to hold the retrieval tool 32D.

The retrieval net 51 has a mesh that permits the waste 42 to pass therethrough but not the capsule 3 to pass therethrough.

As shown in Fig. 6, the retrieval net 51 is formed by netting fine wire 56 along a circular frame 55 in a grid configuration in a manner such that the retrieval net 51 is elastic. The retrieval net 51 takes the shape of a bowl with its own gravity. As shown in Fig. 5, the retrieval net 51 easily catches the capsule 3 in the bowl shape portion thereof is hard to drop the capsule 3.

The subject places the retrieval tool 32D to the toilet bowl 53 when defecating after swallowing the capsule 3. The retrieval tool 32D is installed with the handle 52 hung on the upper edge of the toilet bowl 53 and the circular portion of the retrieval net 51 covering the drain hole 54.

If the capsule 3 is discharged together with the waste 42 in defecation, the capsule 3 is caught and remains in the retrieval net 51 while the waste 42 falls downward as shown in Fig. 5. If the capsule 3 remains mixed within the waste 42, the toilet bowl 53 is flushed several times to wash only the waste 42 away. Since the retrieval net 51 is bowl-shaped, the capsule 3 is not flushed away together with the waste.

If the capsule 3 is found, the subject shakes sticking waste 42 and water drops off the capsule 3, and stores the capsule 3 together with the retrieval tool 32D in the retrieval bag 33 as shown in Fig. 7. The collapsible retrieval net 51 may be flattened into a unbulky shape in storage. Since the handle 52 is also folded at several points, the capsule 3 and the retrieval tool 32D are compactly stored in the bag 33.

When the capsule 3 and the retrieval tool 32D are introduced together into the bag 33 as shown in Fig. 7, the mouth of the bag 33 is closed. The capsule 3 and the retrieval tool 32D are then sent to a manufacturer or a hospital or a collection company. A person in charge in the hospital transfers the retrieved capsules 3 in lot to the manufacturer or the collection company.

The manufacturer or the collection company cleans, sterilizes, disassembles, and discards the capsule 3. Recyclable elements within the capsule 3 may be recycled, and the remaining components may be discarded.

The second example provides the following advantages.

The retrieval net 51 catches the capsule 3, thereby preventing the capsule 3 from being erroneously flushed away.

Since the toilet bowl 53 is flushed prior to the retrieval of the capsule 3, the capsule 3 is retrieved after the waste 42 is washed away. The capsule 3 is retrieved in the clean state thereof.

The retrieval tool 32D is installed with the handle 52 simply hung on the upper edge of the toilet bowl 53. The capsule 3 is easily retrieved. The toilet bowl 53 is not limited to any particular type (The retrieval tool 32D is applied to any type of widely available toilet bowls).

Fig. 8 illustrates a retrieval device 31E containing a retrieval tool 32E in accordance with a first modification of the second example.

In the retrieval device 31E, a retrieval net 51 and a handle 52, as the retrieval tool 32E, are attached to a retrieval bag 33'. The bag 33' has a closing string 58 to close the mouth thereof.

More specifically, the bag 33' has a slit on the bottom portion thereof opposite from the mouth thereof. The handle 52 is inserted through the slit and the end of the handle 52 is connected to the retrieval net 51. The other end of the handle 52 is positioned within the bag 33'. The slit between the bag 33' and the handle 52 is sealed. Alternatively, the bag 33' may be bonded to the handle 52 at the slit thereof by adhesion to close the slit.

During the retrieval operation, the retrieval device 31E is set as shown in Fig. 8. More specifically, the retrieval net 51 is set within the toilet bowl 53 as shown in Fig. 5, and the proximal end of the handle 52 is positioned within the bag 33'. The user holds the proximal end of the handle 52 with the hand in the bag 33'. The user thus performs the retrieval operation with the user's hand kept away from the waste.

When the capsule 3 is retrieved, the user sets the bag 33' inside out to store the capsule 3 together with the retrieval net 51. As shown in Fig. 9, the bag 33' is closed by fastening the mouth string 58.

The first modification of the second example has the following advantages.

Since the retrieval tool 32E is attached to the bag 33', the retrieval device 31E is easy to handle.

After retrieving the capsule 3, the bag 33' is quickly closed in a hygienic way.

A second modification of the second example will now be discussed.

In the second modification, the retrieval net 51 is formed by a magnet or magnetic metal. During the retrieval operation, the subject easily catches the capsule 3. Once caught, the capsule 3 is less subject to falling. The retrieval operation is thus reliably performed. The magnet may be an electromagnet. The electromagnet may be turned on during the retrieval operation only.

### Third Example

A third example will now be discussed with reference to Figs. 10 through 13.

In the third example, a toilet seat type retrieval device includes catching means attached to a toilet bowl. A retrieval tool and capsule storage means are separate elements.

Figs. 10 and 11 show a retrieval tool 32F of the third example. The retrieval tool 32F includes a driver 63 arranged on the toilet seat 62, a bar-like or thin plate rod 64 with one end connected to the driver 63, and a retrieval net 51 connected to the other end of the rod 64. The retrieval net 51 covers a drain hole 65 of the toilet bowl 61. The retrieval net 51 is identical in structure to the one used in the second example.

The driver 63 arranged on the toilet seat 62 includes a vibration motor 76 (see Fig. 13) to vertically vibrate the rod 64 connected to the retrieval net 51. With the driver 63 in operation, the rod 64 vibrates vertically as represented by an arrow in Fig. 10. These elements constitute washing means.

A switch 66 is arranged on the side surface of the toilet seat 62 to switch on and off the vibration motor.

A retrieval device of the third example includes a capsule container 67 shown in Fig. 11 and Figs. 12A-12C.

The capsule container 67 includes a pair of openable holder cups 68, and a watertight storage bag 69.

The holder cups 68 are opened by handles 70. A spring 71 loaded between the handles 70 normally closes the holder cups 68.

The holder cups 68 have a plurality of holes that allow water to pass therethrough but do not allow the capsule 3 to pass therethrough.

The watertight storage bag 69 has a watertight function to prevent the arm and the hand of the user, such as the subject, from being soiled when the user takes up the capsule 3 from the retrieval net 51.

As shown in Fig. 11, the user inserts the hand into the watertight bag 69 to grip the handles 70 of the holder cups 68, opens the holder cups, and catches the capsule 3 from the retrieval net 51. After closing the holder cups 68, the user shakes water off the holder cups 68 through the holes.

The user then sets the watertight bag 69 inside out, stores the holder cups, and closes the watertight bag 69.

Referring to Fig. 12A, the user applies force on the handles 70 to open the holder cups 68 to catch the capsule 3. Releasing the handles 70, the user sets the watertight bag 69 inside out through the mouth thereof as shown in Fig. 12B, stores the holder cups 68 within the watertight bag 69 as shown in Fig. 12C, and closes the mouth of the watertight bag 69.

The subject uses the toilet bowl 61 with the retrieval tool 32F mounted on the toilet seat 62 as shown in Fig. 10 when the subject defecates after swallowing the capsule 3. The toilet bowl 61 having the retrieval tool 32F mounted on the toilet seat 62 may be installed in a hospital or a medical examination center. The retrieval tool 32F may be integrated with the toilet bowl 61.

When the capsule 3 is discharged together with the waste 42, the capsule 3 is caught in the retrieval net 51. If the capsule 3 remains mixed within the waste 42, the toilet bowl 53 is flushed several times to wash only the waste 42 away.

If the capsule 3 is found, the subject turns on the switch 66 of the toilet seat 62 to operate the vibration motor 76. The retrieval net 51 is vibrated together with the capsule 3. The waste 42 sticking to the capsule 3 is thus washed away.

After the waste 42 is washed away, the subject stops the vibration motor 76. The subject takes up the capsule 3 using the capsule container 67 and stores the capsule 3 into the bag 69.

A person in charge collects the bag 69 having the capsule 3 stored therewithin in a hospital or a medical examination center. The collected capsule 3 is then transferred to the manufacturer or the collection company.

The third example has the following advantages.

Since the waste 42 sticking to the capsule 3 is reliably washed away due to vibration of the vibration motor 76, the capsule 3 is retrieved more hygienically.

The user including the subject retrieves the capsule 3 hygienically without touching the toilet bowl 61.

Fig. 13 illustrates an electrical system of a retrieval tool 32G with a sensor in accordance with a first modification of the third example. In the first modification, a metal detector 75 as detection means for detecting the capsule 3 is arranged in addition to the retrieval tool 32F illustrated in Figs. 10 and 11. When the metal detector 75 detects the capsule 3, the vibration motor 76 in the driver 63 operates, thereby automatically washing the capsule 3.

The metal detector 75 detects a metal such as the button battery 21 in the capsule 3. For example, the frame of the retrieval net 51 shown in Fig. 10 forms a search coil 77. A signal line connected to both terminals of the search coil 77 extends through the rod 64 and is coupled to an oscillator 78.

The oscillator 78 together with the search coil 77 forms a resonance circuit, and changes the resonance frequency thereof when a metal approaches the search coil 77.

A resonance voltage with the resonance circuit resonating at the resonance frequency with no metal detected starts decreasing when a metal approaches the search coil 77. The resonance voltage is detected by a voltage detector 79. A comparator (not shown) compares the resonance voltage with a predetermined voltage. When the resonance voltage becomes smaller than the predetermined voltage, the voltage detector 79 outputs a signal representing the detection of metal of the capsule 3 to a CPU 80 as control means in the driver 63. In response to the metal triggered signal, the CPU 80 causes the vibration motor 76 to vibrate.

In this case, after detecting the capsule 3, the CPU 80 also works as a timer to continuously switch on the vibration motor 76 for a predetermined period of time (30 seconds, for example).

After vibration for the predetermined period of time, the CPU 80 causes a loudspeaker 81 to sound a beep, a melody, a voice to notify the subject that the capsule 3 is discharged or that the discharged capsule 3 is washed.

When the sound is emitted, the subject simply flushes the toilet and does not need to observe the subject's own waste 42 (Toilet flushing may be automatically performed).

The first modification of the third example provides the following advantages. Since the detection, vibration, and washing of the capsule 3 are automatically performed, the retrieval operation is easily and hygienically performed. The subject is free from observing the user's own waste 42 to look for the capsule 3.

Since the search coil 77 is arranged on the retrieval net 51, the discharged capsule 3 is reliably detected.

If the capsule 3 is detected, the vibration motor 76 automatically washes the capsule 3. The subject can learn the discharging of the capsule 3 without the need for using the loudspeaker 81. The vibration motor 76 is thus used as alerting means.

If the capsule 3 contains a retrieval detection magnet, a magnetic sensor 85 (or a gauss meter) shown in Fig. 14 is used instead of the metal detector 75.

As shown in Fig. 14, wire (coil) 86 is contained in an external frame 51' (of the retrieval net 51 shown in Fig. 10). When the capsule 3 containing the magnet falls into the retrieval net 51, an induced electromotive force is generated in the wire 86. A current detector 87 detects the resulting current, and outputs a detection output to the CPU 80 in the driver 63. As shown in Fig. 13, the vibration motor 76 is operated.

A second modification of the third example will now be discussed.

The detection of the capsule 3 may be alerted to the subject using light like flash, or a combination of sound and light rather than the sound only in the first modification. As a result, even a person having hearing difficulty (such as a person of advanced age) may sense the alerting.

In another modification, an ultrasonic transducer may be used as washing means instead of the vibration motor 76. The waste 42 is thus reliably washed away.

### Fourth Example

A fourth example will now be discussed with reference to Figs. 15 and 16. The fourth example relates to a toilet dedicated to a capsule retrieval for use in a hospital or a medical examination center.

As shown in Fig. 15, a capsule detection sensor 93 is arranged near a drain hole 92 and a capsule retrieval net 95 is arranged in a drainage 94 in a toilet bowl 91 installed in a hospital or the like.

Close to the capsule retrieval net 95, a retrieval lid 96 is arranged to close a retrieval hole through which the capsule 3 is retrieved. By removing the retrieval lid 96, the capsule 3 caught in the capsule retrieval net 95 is retrieved.

The subject may take a dose of purgative after swallowing the capsule 3. After a medical examination (namely, about 8 hours after the swallowing of the capsule 3 or after the moment no signal is received from the capsule 3), the subject may defecate in the toilet bowl 91.

If the capsule detection sensor 93 detects a metal or a magnet in the capsule 3, a lamp 97 flashes to alert the subject to the discharged capsule 3. The subject may flush the toilet bowl 91 and leave from there.

After retrieval, staff in the hospital, the medical examination center, or the collection company cleans, sterilizes, discards, or recycles the capsule 3 discharged from the toilet bowl 91 with the retrieval lid 96 pivoted.

Collection may be performed in lot after some amount of capsules 3 is accumulated.

The fourth example provides the following advantages.

The subject remains at ease during the medical examination because the subject is freed from observing the capsule 3 discharged by him or her, and retrieving the capsule 3.

Since the toilet bowl 91 is flushed prior to the retrieval of the capsule 3, the capsule 3 is retrieved after the waste 42 is washed away. The capsule 3 is retrieved in the clean state thereof.

A plurality of capsules 3 are retrieved together.

A first modification of the fourth example will now be discussed.

The first modification employs no capsule detection sensor 93. Sometime after the completion of medical examination or after the subject takes a predetermined dose of purgative (or intestinal irrigation) in succession to the completion of medical examination, the subject defecates several times in the toilet bowl 91. Depending on the dose of purgative, or time elapse from the medical examination, the capsule 3 may be discharged.

Without the capsule detection sensor 93, the toilet bowl 91 is simplified with costs thereof reduced.

A second modification of the fourth example will now be discussed with reference to Fig. 16.

The difference between a capsule retrieval device 101 shown in Fig. 16 and the toilet bowl 91 shown in Fig. 15 is that the capsule retrieval net 95 and a pivotal lid 96 are integrally pivoted. The discharging of the capsule 3 is detected, and the capsule 3 is flushed together with the waste 42 to the drainage 94 in response to the flushing operation. The capsule 3 is caught in the capsule retrieval net 95.

The capsule detection sensor 93 detects the capsule 3. After time elapse set to be longer than flushing operation using a timer (not shown), a motor (not shown) pivots the capsule retrieval net 95 and the pivotal lid 96 about a rotary axis 102 in response to the output from the capsule detection sensor 93.

Along with the pivotal motion, the capsule 3 drops down from the capsule retrieval net 95 (the pivotal lid 96 represented by two-dot-and-dash chain line in the pivoted position thereof).

The capsule 3 caught by the capsule retrieval net 95 drops into a retrieval pipe 103. The capsule 3 is automatically washed by an automatic washing device 105, automatically sterilized by an automatic sterilization device 106, automatically dried by an automatic drying device 107, and then automatically packaged by an automatic packaging device 108. A packaged capsule 3 is collected by a collection company. The automatic washing device 105 may be arranged below a floor surface 109 where the toilet bowl 91 is installed.

The second modification of the fourth example permits hygienic retrieval operation because the user is free from directly touching the capsule 3.

A third modification of the fourth example is discussed with reference to Figs. 16 and 17. The difference between a capsule 3B shown in Fig. 17 and the capsule 3 shown in Fig. 2 is that the capsule 3B includes a non-volatile memory 111 such as a flash memory. The capsule 3B also contains an optical sensor 112 that detects light from the outside. A signal detected by the optical sensor 112 is output to the control circuit 19.

The control circuit 19 determines whether light is detected during light emission suspension period of the white LED 18 that emits at intervals. If light is detected, the capsule 3B is determined as being discharged from within the body of the subject. Images captured by the capsule 3B are stored in the memory 111. When the capsule 3B is discharged from within the body of the subject, the control circuit 19 receives the signal from the optical sensor 112. Under the control of the control circuit 19, image data stored in the memory 111 is sent from the antenna 23. During the passing of the capsule 3B through the tracts of the subject body, the capsule 3B performs image capturing only, and does not transmit data outward.

The capsule retrieval device 101 shown in Fig. 16 includes a signal reading device 110 represented by two-dot-and-dash chain line staged in succession to the automatic drying device 107. The signal reading device 110 receives the image data transmitted by the capsule 3. The image data is then stored in an image database (server) managed by a hospital, a medical examination center, or a manufacturer. Physicians may access the database (server) to diagnose the subject viewing the images.

The position of the signal reading device 110 is not limited to a stage subsequent to the automatic drying device 107. The signal reading device 110 may be installed at a prior stage, or may be installed within the toilet bowl. The image pickup device (the CMOS imager 17) may be used as the optical sensor 112. In this case, the component count of the device is reduced.

In accordance with the third modification of the fourth example, the retrieval and washing of the capsule 3 and data collection are automatically performed in a coordinated fashion. The retrieval operation is thus performed efficiently without human intervention.

A fourth modification of the fourth example will now be discussed. Fig. 18 illustrates a capsule 3C of the fourth modification.

The difference between the fourth modification and the third modification is that an infrared emitting element 123 such as a light emitting diode is substituted for the antenna 23 in the capsule 3B shown in Fig. 2. The image data stored in the memory 111 is optically transmitted from the infrared emitting element 123 after the capsule 3C is discharged from within the body of the subject.

The fourth modification of the fourth example employs a receiving system including an infrared photodetector instead of the signal reading device 110 represented by two-dot-and-dash chain line arranged on the capsule retrieval device 101 shown in Fig. 16. The receiving system receives the image data transmitted by the capsule 3. The image data is then stored in an image database (server) managed by the hospital, the medical examination center, or the manufacturer. Physicians may access the database server to diagnose the subject viewing the images.

In accordance with the fourth modification of the fourth example, the retrieval and washing of the capsule 3 and data collection are automatically performed in a coordinated fashion. The retrieval operation is thus performed efficiently without human intervention.

### First Embodiment

A first embodiment of the present invention will now be discussed with reference to Fig. 19. Fig. 19 illustrates an electrical system of a retrieval tool 32H of the first embodiment. The first embodiment of the present invention includes detecting means 131 which detects the capsule 3 by receiving a weak radio signal emitted from the capsule 3. The detecting means 131 is arranged on the toilet seat 62. More specifically, the detecting means 131 is arranged in the driver 63 attached to the toilet seat 62 and the rod 64 shown in Fig. 11.

The detecting means 131 includes an antenna 132 for detecting a radio wave from the capsule 3, a resonance and detector circuit 133 as received signal intensity detection means connected to the antenna 132, and an amplifier 134 for amplifying a detected signal. The output of the amplifier 134 is fed to the CPU 80.

Since the radio wave signal from the capsule 3 is too weak to be detected with the capsule 3 staying in the body, the detecting means 131 is unable to receive the radio wave signal. The radio wave signal becomes strong enough to be received by the detecting means 131 when the capsule 3 is discharged from within the body of the subject. When a signal in level higher than a noise level is detected, the CPU 80 determines that the capsule 3 is discharged from within the body of the subject.

The detection gain of the detecting means 131 may be increased. The CPU 80 compares the signal with a reference value 135 that is set to be higher in level than a signal the detecting means 131 detects when the capsule 3 stays in the subject body. When the signal rises above the reference value 135, the CPU 80 determines that the capsule 3 is discharged from within the subject body.

When a signal higher in level than the reference value 125 is input, the CPU 80 reports through the loudspeaker 81 that the capsule 3 has been discharged from within the subject body. The CPU 80 may reports through the loudspeaker 81 after driving the vibration motor 76 as already discussed with reference to Fig. 11.

Referring to Fig. 19, the antenna 132 is arranged in the rod 64. Alternatively, the antenna 132 may be arranged along the frame of the retrieval net 51 as shown in Figs. 13 and 14. The detecting means 131 may be arranged close to the rod 64.

A first modification of the first embodiment of the present invention will now be discussed.

Detecting means that detects the capsule 3 by receiving a radio wave signal transmitted from the capsule 3 is arranged in the extra-corporeal unit 5 (see Fig. 1A) the subject wears during the medical examination. The extra-corporeal unit 5 receives a signal transmitted by the capsule 3 during the medical examination. The strength of the signal greatly changes from when the capsule 3 stays in the subject body to when the capsule 3 is discharged from within the subject body.

The extra-corporeal unit 5 monitors the difference in the signal strength, thereby detecting that the capsule 3 is discharged from the subject body. The CPU 80 alerts the subject to the discharging of the capsule 3 using a buzzer, vibration, a melody, sound, light emission of an LED, an EL device, an electric bulb, a display by an electric bulletin board or a liquid-crystal monitor.

Fig. 20 illustrates the structure of the extra-corporeal unit 5. A radio wave signal received by an antenna 11 (constituting the antenna unit 4) is input to a receiver circuit 141 for demodulation. The signal processing and control circuit 142 processes the demodulated signal into compressed image data, which is then recorded on a hard disk 143, or into a video signal, which is then displayed on the liquid-crystal monitor 12.

The output of the receiver circuit 141 is also input to a signal strength detector circuit 144. The signal strength detector circuit 144 detects the signal input from the receiver circuit 141, compares the strength of the signal with a reference value 145, and sends the result of comparison to the signal processing and control circuit 142. If the signal received from the receiver circuit 141 is higher than the reference value 145, the signal processing and control circuit 142 alerts the subject to the discharging of the capsule 3 sounding a buzzer 13, or blinking a portion of a screen of the liquid-crystal monitor 12. An LED also may be used to alert the subject, as already discussed.

The subject may flush the toilet and leave there. Since the reception function of the extra-corporeal unit 5 is used, no particular detection means is required on the toilet side. The cost of the toilet is reduced.

A second modification of the first embodiment of the present invention will now be discussed.

In the second modification, the signal that is transmitted at the discharge of the capsule is not an standard examination signal. After a predetermined period of time (for example, 10 hours later) from the start of the capsule operation, a timer causes the capsule to shift to a discharge alerting simple signal transmission mode. The transmission of a simple signal consumes less power, and the capsule can continuously transmit the simple signal in the subject body for a long period of time.

In the above-referenced examples and embodiments, the capsule type endoscopes having an illumination and image pickup means have been discussed as the medical capsule. The present invention is not limited to the capsule type endoscopes 3 or the like. The medical capsule may include the following means (a)-(c).

### (a) Sensor Means

The capsule has, on the external surface, a variety of sensors including an optical sensor, a fluorescence sensor, a pH sensor, a temperature sensor, a pressure sensor, an acceleration sensor, and a blood sensor (a hemoglobin sensor). The sensors are mounted on the capsule so that watertightness is assured with the sensing portion of each sensor exposed to the external surface of the capsule.

The sensing portion captures biomedical information including light level in the body, a chemical level (pH value) of internal fluid, a temperature of each organ, a pressure acting on the external surface of the capsule when the capsule passes through the tracts of the body, and a hemoglobin level at each organ (presence or absence of bleeding). Through communication means, the biomedical information is transmitted to receiving means arranged outside the subject body.

Data received by the receiving means is compared with reference values. Physicians and co-medicals diagnose the subject based on the information concerning the presence or absence of illness and bleeding, and a present position and passing state of the capsule. The medical capsule measures the pH level and the hemoglobin level in the digestive tracts of the body without any pain to the subject. Physicians diagnose the subject in terms of digestive tract illness and perform biomedical examination. The capsule may be selectively equipped with a plurality of sensors for efficient examination.

### (b) Ultrasonic Probe

An ultrasonic probe is attached to the capsule in a watertight manner so that an acoustic lens of the ultrasonic probe is exposed to the external surface of the capsule.

An ultrasonic diagnosing image of an internal tract is acquired from an ultrasonic transceiver circuit within the capsule. Resulting data is transmitted to the extra-corporeal receiving means in the manner already discussed. An in-depth region of a tract, such as of the small intestine, deep from the surface thereof may be examined to determine the presence or absence of an abnormal lesion. Together with visual observation means, physicians diagnose the surface and the in-depth region of the tract at the same time.

### (c) Medical Treatment Means

The capsule has an entrance leading to a medicine storage compartment and a bodily fluid storage compartment. The entrance is closed by soluble membrane, such as gelatin that is digested by gastric juice, or fatty membrane that is digested by intestinal juice.

When the capsule reaches a target location, a medicine may be directly administered, or a bodily fluid may be sampled.

After verifying a bleeding location using the blood sensor and the visual observation means, the physician instructs the capsule to operate a treatment tool such a syringe needle for a hemostatic drug. The hemostatic drug such as ethanol or powder medicine is thus sprayed onto a bleeding area of the tract for hemostasis.

A part of or the entire above-referenced embodiment falls within the scope of the present invention.

### Industrial Applicability

As described above, the medical capsule retrieval device of the present invention detects and catches the medical capsule having biomedical information collected within the human body when the medical capsule is discharged from within the human body. The medical capsule retrieval device is thus appropriate for retrieving the discharged medical capsule.

## Claims

1. A medical capsule retrieval device for retrieving a medical capsule (3, 3B, 3C) adapted to emit a radio wave signal, comprising
catching means for catching the medical capsule (3, 3B, 3C) discharged from within a human body, wherein
the catching means comprises a net (51, 95) for retrieving or catching the medical capsule (3, 3B, 3C),
**characterized in that** the medical capsule retrieval device further comprises detecting means comprising receiving means (131) that detects the radio wave signal emitted from the medical capsule (3, 3B, 3C).

2. The medical capsule retrieval device according to claim 1, wherein the detecting means comprises the receiving means (131) and determining means (80) for determining the discharging of the medical capsule (3, 3B, 3C) from within the human body based on a variation in the intensity of the signal received by the receiving means (131).

3. The medical capsule retrieval device according to one of claims 1 or 2, further comprising:
alerting means for alerting the detection of the medical capsule(3, 3B, 3C).

4. The medical capsule retrieval device according to claim 3, wherein the alerting means comprises vibration generating means (76).

5. The medical capsule retrieval device according to claim 3, wherein the alerting means comprises light emitting means including one of a LED, an EL device, and an electric bulb.

6. The medical capsule retrieval device according to claim 3, wherein the alerting means comprises display means such as a liquid crystal display.

7. The medical capsule retrieval device according to claim 3, wherein the alerting means comprises sound generating means (81) for generating a sound such as a buzzer or a melody.

8. The medical capsule retrieval device according to any one of claims 1 to 7,
wherein the radio wave signal comprises examination data acquired from the medical capsule (3, 3B, 3C).

9. The medical capsule retrieval device according to any one of claims 1 to 7, wherein the radio wave signal comprises image data.

10. A retrieval method for retrieving a medical capsule (3, 3B, 3C) adapted to emit a radio wave signal, comprising:
a step of catching the medical capsule (3, 3B, 3C) discharged from within a human body using catching means, wherein
the catching means is a net (51, 95) for retrieving or catching the medical capsule (3, 3B, 3C),
**characterized in that** the retrieval method further comprises a step of
detecting the medical capsule (3, 3B, 3C) by detecting the radio wave signal emitted from the medical capsule (3, 3B, 3C) by receiving means.

11. The retrieval method according to claim 10, further comprising a step of alerting the detection of the medical capsule (3, 3B, 3C) using alerting means.

## Patentansprüche

1. Bergevorrichtung für eine medizinische Kapsel zum Bergen einer medizinischen Kapsel (3, 3B, 3C), die dazu eingerichtet ist, ein Funkwellensignal auszusenden, umfassend
ein Fangmittel zum Fangen der aus dem Inneren eines menschlichen Körpers abgeführten medizinischen Kapsel (3, 3B, 3C), wobei
das Fangmittel ein Netz (51, 95) zum Bergen oder Fangen der medizinischen Kapsel (3, 3B, 3C) umfasst,
**dadurch gekennzeichnet, dass** die Bergevorrichtung für eine medizinische Kapsel ferner ein Erfassungsmittel umfasst, das ein Empfangsmittel (131) umfasst, welches das von der medizinischen Kapsel (3, 3B, 3C) ausgesandte Funkwellensignal erfasst.

2. Bergevorrichtung für eine medizinische Kapsel nach Anspruch 1, wobei das Erfassungsmittel das Empfangsmittel (131) und Bestimmungsmittel (80) zum Bestimmen des Abführens der medizinischen Kapsel (3, 3B, 3C) aus dem Inneren des menschlichen Körpers auf der Grundlage einer Veränderung der Intensität des von dem Empfangsmittel (131) empfangenen Signals umfasst.

3. Bergevorrichtung für eine medizinische Kapsel nach einem der Ansprüche 1 oder 2, ferner umfassend:
ein Meldemittel zum Melden des Erfassens der medizinischen Kapsel (3, 3B, 3C).

4. Bergevorrichtung für eine medizinische Kapsel nach Anspruch 3, wobei das Meldemittel Vibrationserzeugungsmittel (76) umfasst.

5. Bergevorrichtung für eine medizinische Kapsel nach Anspruch 3, wobei das Meldemittel Lichtemissionsmittel einschließlich eines von einer LED, einer EL-Vorrichtung und einer elektrischen Lampe umfasst.

6. Bergevorrichtung für eine medizinische Kapsel nach Anspruch 3, wobei das Meldemittel Anzeigemittel, wie eine Flüssigkristallanzeige, umfasst.

7. Bergevorrichtung für eine medizinische Kapsel nach Anspruch 3, wobei das Meldemittel Geräuscherzeugungsmittel (81) zum Erzeugen eines Geräusches, wie ein Summen oder eine Melodie, umfasst.

8. Bergevorrichtung für eine medizinische Kapsel nach einem der Ansprüche 1 bis 7, wobei das Funkwellensignal von der medizinischen Kapsel (3, 3B, 3C) erfasste Untersuchungsdaten umfasst.

9. Bergevorrichtung für eine medizinische Kapsel nach einem der Ansprüche 1 bis 7, wobei das Funkwellensignal Bilddaten umfasst.

10. Ein Bergeverfahren zum Bergen einer medizinischen Kapsel (3, 3B, 3C), die dazu eingerichtet ist, ein Funkwellensignal auszusenden, umfassend:
einen Schritt eines Fangens der aus dem Inneren eines menschlichen Körpers abgeführten medizinischen Kapsel (3, 3B, 3C) unter Verwendung eines Fangmittels, wobei
das Fangmittel ein Netz (51, 95) zum Bergen oder Fangen der medizinischen Kapsel (3, 3B, 3C) ist,
**dadurch gekennzeichnet, dass** das Bergeverfahren ferner einen Schritt eines Erfassens der medizinischen Kapsel (3, 3B, 3C) durch Erfassen des von der medizinischen Kapsel (3, 3B, 3C) ausgesandten Funkwellensignals anhand von Empfangsmitteln umfasst.

11. Bergeverfahren nach Anspruch 10, ferner umfassend einen Schritt eines Meldens des Erfassens der medizinischen Kapsel (3, 3B, 3C) unter Verwendung von Meldemitteln.

## Revendications

1. Dispositif de récupération de capsule médicale pour récupérer une capsule (3, 3B, 3C) médicale adaptée à émettre un signal d'onde radio, comprenant :
un moyen de capture pour capturer la capsule (3, 3B, 3C) médicale déchargée de l'intérieur d'un corps humain, dans lequel
le moyen de capture comprend un filet (51, 95) pour récupérer ou capturer la capsule (3, 3B, 3C) médicale,
**caractérisé en ce que** le dispositif de récupération de capsule médicale comprend en outre
un moyen de détection comprenant un moyen de réception (131) qui détecte le signal d'onde radio émis de la capsule (3, 3B, 3C) médicale.

2. Dispositif de récupération de capsule médicale selon la revendication 1, dans lequel le moyen de détection comprend le moyen de réception (131) et un moyen de détermination (80) pour déterminer la décharge de la capsule (3, 3B, 3C) médicale de l'intérieur du corps humain sur la base d'une variation de l'intensité du signal reçu par le moyen de réception (131).

3. Dispositif de récupération de capsule médicale selon l'une des revendications 1 ou 2, comprenant en outre :
un moyen d'alerte pour alerter de la détection de la capsule (3, 3B, 3C) médicale.

4. Dispositif de récupération de capsule médicale selon la revendication 3, dans lequel le moyen d'alerte comprend un moyen (76) de génération de vibration.

5. Dispositif de récupération de capsule médicale selon la revendication 3, dans lequel le moyen d'alerte comprend un moyen électroluminescent incluant un parmi une LED, un dispositif EL, et une ampoule électrique.

6. Dispositif de récupération de capsule médicale selon la revendication 3, dans lequel le moyen d'alerte comprend un moyen d'affichage tel qu'un afficheur à cristaux liquides.

7. Dispositif de récupération de capsule médicale selon la revendication 3, dans lequel le moyen d'alerte comprend un moyen (81) de génération de son pour générer un son tel qu'un vibreur ou une mélodie.

8. Dispositif de récupération de capsule médicale selon l'une quelconque des revendications 1 à 7, dans lequel le signal d'onde radio comprend des données d'examen acquises à partir de la capsule (3, 3B, 3C) médicale.

9. Dispositif de récupération de capsule médicale selon l'une quelconque des revendications 1 à 7, dans lequel le signal d'onde radio comprend des données d'images.

10. Procédé de récupération pour récupérer une capsule (3, 3B, 3C) médicale adaptée à émettre un signal d'onde radio, comprenant :
une étape de capture de la capsule (3, 3B, 3C) médicale déchargée de l'intérieur d'un corps humain en utilisant un moyen de capture, dans lequel
le moyen de capture est un filet (51, 95) pour récupérer ou capturer la capsule (3, 3B, 3C) médicale,
**caractérisé en ce que** le procédé de récupération comprend en outre une étape de
détection de la capsule (3, 3B, 3C) médicale en détectant le signal d'onde radio émis de la capsule (3, 3B, 3C) médicale par un moyen de réception.

11. Procédé de récupération selon la revendication 10, comprenant en outre une étape d'alerte de la détection de la capsule (3, 3B, 3C) médicale en utilisant un moyen d'alerte.
